# EUROPÄISCHE PATENTSCHRIFT

(11) **EP 2 424 491 B1**
(45) Veröffentlichungstag und Bekanntmachung des Hinweises auf die Patenterteilung: **24.05.2017**
(21) Anmeldenummer: 10713870.3
(22) Anmeldetag: 11.03.2010
(51) Int. Cl.: A61K 8/22, A61Q 5/06, A61K 8/81

(54) **FILMBILDNER IN HAARFARBEN**
FILM FORMER IN HAIR COLOURS
AGENT FILMIFIANT DANS DES COMPOSITIONS COLORANTES POUR LES CHEVEUX

(30) Priorität: 29.04.2009 DE 102009002729
(43) Veröffentlichungstag der Anmeldung: 07.03.2012
(73) Patentinhaber: Henkel AG & Co. KGaA, 40589 Düsseldorf (DE)
(72) Erfinder: WITTE, Christiane, 25491 Hetlingen (DE); MANNECK, Hartmut, 23860 Klein Wesenberg (DE)
(86) Internationale Anmeldenummer: PCT/EP2010/053091
(87) Internationale Veröffentlichungsnummer: WO 2010/124897

(56) Entgegenhaltungen:
- WO-A1-2004/082646
- WO-A1-2009/085838
- WO-A1-2010/015442
- WO-A1-2010/026097
- DE-A1- 2 623 692
- DE-A1- 2 822 358
- FR-A1- 2 820 033
- JP-A- 10 279 442
- US-A1- 2004 025 264
- US-A1- 2004 052 746

## Beschreibung

Die Erfindung betrifft ein Mittel zur Farbveränderung von keratinhaltigen Fasern, insbesondere menschlichen Haaren. Solche Mittel enthalten in einem kosmetischen Träger mindestens eine farbgebende Komponente und mindestens ein kationisierbares, nicht permanent ionisches Copolymer, sowie Wasserstoffperoxid und eine Peroxoverbindung enthält. Weiterhin betrifft die Erfindung ein Verfahren zur Anwendung von solchen Mitteln auf keratinhaltigen Fasern. Darüber hinaus betrifft die Erfindung die Verwendung dieses Mittels zur Verbesserung des Farberhalts. Schließlich betrifft die vorliegende Erfindung eine Mehrkomponentenverpackungseinheit, wobei sich das erfindungsgemäße Mittel durch Vermischen der Einzelkomponenten ergibt.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Als Entwicklerkomponenten werden üblicherweise primäre aromatische Amine mit einer weiteren, in para- oder ortho-Position befindlichen, freien oder substituierten Hydroxy- oder Aminogruppe sowie heterocyclische Amine eingesetzt. Als Kupplerkomponenten werden zumeist m-Phenylendiaminderivate, Naphthole, Pyridinderivate, Resorcin und Resorcinderivate, Pyrazolone und m-Aminophenole verwendet. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Sollen Substrate neben der Färbung aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Insbesondere oxidative Haarfärbemittel sind trotz ihrer vorteilhaften Färbeeigenschaften für den Anwender mit Nachteilen behaftet. Einerseits führt der Einsatz von Oxidationsmittel und die zur Färbung oder Aufhellung notwendigen, alkalischen Zubereitungen zur Ausfärbung respektive Entwicklung der eigentlichen Färbung zu Schädigungen in der Haarstruktur und auf der Haaroberfläche. Andererseits besteht auch weiterhin ein Verbesserungsbedarf im Bezug auf Farberhalt und Stabilität der Färbungen auf dem Haar.

Sollen ergraute oder weiße Haare gefärbt werden, besteht außerdem weiterhin Bedarf nach Färbemittel, die eine effiziente Grauabdeckung erwirken. Gleichzeitig sollen die Mittel unterschiedlich vorbehandeltes Haar gleichmäßig färben und daher über ein sehr gutes Egalisiervermögen verfügen.

In US 2004/0052746 A1 werden Polymere beansprucht, die durch Polymerisation mindestens eines amino-substituierten Monomers, mindestens eines hydrophoben nichtionischen Vinyl-Monomers, mindestens eines assoziativen Vinyl-Monomers und mindestens eines semi-hydrophoben Vinyl-Monomers erhalten werden. Ein weiterer Anspruch der Schrift zielt auf Haarbehandlungsformulierungen ab, welche diese Polymere enthalten.

JP 10279442 A offenbart ein Haarkosmetikum enthaltend einen kationischen Verdicker, direktziehende Farbstoffe und verschiedene Festigungspolymere. Bei den festigenden Polymeren kann es sich auch um kationische Verdicker auf Basis von aminosubstituierten Vinylmonomeren handeln.

WO 2004/082646 A1 adressiert ein Verfahren zur Minimierung des Farbverlustes von Haaren unter Anwendung von färbenden pflanzlichen Phenolen. In den Beispielen wird auch der Einsatz von amino-alkyl substituierten Acrylamid-Polymeren offenbart.

DE 2623692 A1 beschreibt Färbemittel auf Basis von Oxidaitonsfarbstoffvorprodukten, welche Copolymere aus Vinylpyrrolidon, Dimethylaminoethylmethacrylat und Polyethylenglycol enthalten.FR2820033 A1 beschreibt Mittel zur direkten Haarfärbung enthaltend Copolymere basierend auf Vinyllactam-Monomeren (bevorzugt Vinylpyrrolidon), welche kationisch oder kationisierbar sind, unter anderem zur aufhellenden Färbung.

In nicht vorhersehbarer Weise wurde nun gefunden, dass farbverändernde Mittel hinsichtlich ihres Egalisiervermögens und ihres Vermögen zur Grauabdeckung, besonders jedoch im Bezug auf den Farberhalt signifikant verbessert werden können, wenn sie ein spezifisches kationisierbares Copolymer mit einer basischen Aminoalkyl-Seitenkette enthalten.

Ein erster Gegenstand der vorliegenden Erfindung ist daher ein Mittel zur Farbveränderung von keratinischen Fasern, insbesondere menschlichen Haaren, enthaltend in einem kosmetischen Träger mindestens einen direktziehenden Farbstoff, ausgewählt aus der Gruppe, gebildet aus Nitrofarbstoffen, kationischen direktziehenden Farbstoffen und anionischen direktziehenden Farbstoffen, dadurch gekennzeichnet, dass das Mittel zusätzlich ein nicht permanent ionisches Copolymer enthält, welches sich von mindestens einem kationisierbaren Monomer der allgemeinen Formel (F1), 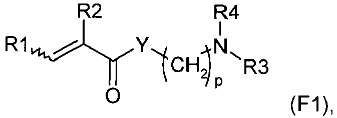 worin R1 für Wasserstoff, R2, R3 und R4 jeweils für eine Methylgruppe, Y für eine NH-Gruppe und p für die Zahl 3 steht, und von N-Vinyl-pyrrolidin-2-on ableitet und zusätzlich Wasserstoffperoxid und als weiteres chemisches Oxidationsmittel eine feste Peroxoverbindung enthält, welche ausgewählt wird aus der Gruppe, die gebildet wird aus Ammoniumperoxodisulfat, Kaliumperoxodisulfat, Natriumperoxodisulfat, Ammoniumperoxomonosulfat, Kaliumperoxomonosulfat, Natriumperoxomonosulfat, Kaliumperoxodiphosphat, Magnesiumpercarbonat, Bariumperoxid, Natriumperborat sowie Harnstoffperoxid und Melaminperoxid.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.
Ein wässriger Träger enthält im Sinne der Erfindung mindestens 30 Gew.-%, insbesondere mindestens 50 Gew.-% Wasser, bezogen auf das Gesamtgewicht der Anwendungsmischung.
Als ersten wesentlichen Inhaltsstoff enthält das erfindungsgemäße Mittel mindestens ein nicht permanent ionisches Copolymer, welches sich ableitet von mindestens einem kationisierbaren Monomer der allgemeinen Formel (F1) 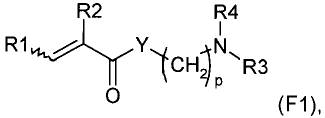 worin R1 für Wasserstoff, R2, R3 und R4 jeweils für eine Methylgruppe, Y für eine NH-Gruppe und p für die Zahl 3 steht.

Der Begriff "permanent ionisch" ist dabei so zu verstehen, dass das Copolymer eine permanente Ladung trägt, wie sie beispielsweise durch Alkylierung eines basischen, tertiären Stickstoffs erfolgen kann. Copolymere, die sich von Monomeren gemäß Formel (F1) und mindestens einem weiteren Monomeren, welches eine permanente Ladung trägt, ableiten, sind daher im Rahmen der vorliegenden Anmeldung nicht erfindungsgemäß. Insbesondere sind daher permanent kationische Polymere, wie zum Beispiel Polyquaternium-55, nicht von Umfang der vorliegenden Anmeldung umfasst.

"Nicht permanent ionische" Copolymere liegen im Gegensatz dazu nicht über den gesamten, physiologisch verträglichen pH-Bereich geladen vor, sondern stehen in einem vom pH-Wert abhängigen Gleichgewicht mit ihrer ungeladenen, nicht ionischen Form.

Der Begriff "kationisierbar" ist im Sinne der Anmeldung zu verstehen, dass die Verbindung einen basischen Stickstoff enthält, welcher unter physiologisch verträglichen pH-Bedingungen befähigt ist, protoniert zu werden und damit in eine kationische Form überzugehen. Je nach pH-Wert wird dabei die protonierte, kationische oder die basische, ungeladene Form überwiegen.

Eine erfindungsgemäße Ausführungsform liegt dann vor, wenn das Mittel der vorliegenden Anmeldung ein Copolymer enthält, das sich mindestens von einem kationisierbaren Monomer der allgemeinen Formel (F1) ableitet, worin R1 für Wasserstoff, R2, R3 und R4 jeweils für eine Methylgruppe, Y für eine NH-Gruppe und p für die Zahl 3 steht :Methacrylsäure-3-(dimethylaminopropyl)-amid.

Erfindungsgemäße Copolymere zeichnen sich dadurch aus, dass sie sich als weiterem Monomer von mindestens einem N-Vinyl-Lactam ableiten, ausgewählt aus N-Vinyl-pyrrolidin-2-on

Beispiele für kommerziell erhältliche Copolymere gemäß vorliegender Erfindung werden unter dem Handelsnamen Styleze^{®} CC10 (Vinylpyrrolidinon/ Dimethylaminopropylmethacrylamid Copolymer; ISP) vertrieben.

Kommerziell erhältliche Terpolymere, die ebenfalls erfindungsgemäß einsetzbar sind, werden unter dem Handelsnamen Aquaflex^{®} SF 40 (Vinylpyrrolidinon/ Vinylcaprolactam/ Dimethylaminopropylmethacrylamid Terpolymer; ISP) vertrieben.

Erfindungsgemäße Mittel sind dadurch gekennzeichnet, dass das Copolymer sich mindestens von einem kationisierbaren Monomer der allgemeinen Formel (F1), worin R1 für Wasserstoff, R2, R3 und R4 jeweils für eine Methylgruppe, Y für eine NH-Gruppe und p für die Zahl 3 steht, und mindestens N-Vinyl-pyrrolidin-2-on ableitet.

Erfindungsgemäß vorteilhaft als Copolymere sind solche, die ein Molekulargewicht von 100,000 bis 500,000 g/mol, insbesondere von 250,000 bis 350,000 g/mol besitzen.

In einer weiteren Ausführungsform enthält das erfindungsgemäße Mittel das nicht permanent ionische Copolymer in einer Menge von 0,005 bis 10 Gew.-%, bevorzugt von 0,01 bis 5 Gew.-% und ganz besonders bevorzugt zu 0,05 bis 2 Gew.-%, jeweils bezogen auf das Gesamtgewicht des anwendungsbereiten Mittels.

Als weiteren wesentlichen Inhaltstoff enthält das erfindungsgemäße Mittel mindestens einen direktziehenden Farbstoff aus der Gruppe, gebildet aus Nitrofarbstoffen, kationischen direktziehenden Farbstoffen und anionischen direktziehenden Farbstoffen.

Geeignete direktziehende Nitrofarbstoffe sind
- blaue Nitrofarbstoffe, wie
   1,4-Bis-[(2-hydroxyethyl)amino]-2-nitrobenzol, 1-(2-Hydroxyethyl)amino-2-nitro-4-[di-(2-hydroxyethyl)amino]-benzol (HC Blue 2), 1-Methylamino-4-[methyl-(2,3-dihydroxypropyl)amino]-2-nitrobenzol (HC Blue 6), 1-[(2,3-Dihydroxypropyl)-amino]-4-[ethyl-(2-hydroxyethyl)amino]-2-nitrobenzol Hydrochlorid (HC Blue 9), 1-[(2,3-Dihydroxypropyl)amino]-4-[methyl-(2-hydroxyethyl)amino]-2-nitrobenzol (HC Blue 10), 4-[Di-(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol Hydrochlorid (HC Blue 12), 2-((4-Amino-2-nitrophenyl)amino)-5-dimethylamino-benzoesäure (HC Blue 13), 1-Amino-3-methyl-4-[(2-hydroxyethyl)amino]-6-nitrobenzol (HC Violet 1), 1-(3-Hydroxypropylamino)-4-[di-(2-hydroxyethyl)amino]-2-nitrobenzol (HC Violet 2), 1-(2-Aminoethylamino)-4-[di-(2-hydroxyethyl)amino]-2-nitrobenzol, 4-(Di-(2-hydroxyethyl)amino)-2-nitro-1-phenylaminobenzol;
- rote Nitrofarbstoffe, wie
   1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)amino]benzol, 4-Amino-3-nitrophenol, 4-[(2-Hydroxyethyl)amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 4-(2,3-Dihydroxypropoxy)-1-[(2-hydroxyethyl)amino]-2-nitrobenzol (HC Orange 3), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2-Hydroxyethyl)-amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3-Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, 7-Amino-3,4-dihydro-6-nitro-2H-1,4-benzoxazin (HC Red 14);
- gelbe Nitrofarbstoffe, wie
   1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxy-ethyl)amino]-5-nitrobenzol (HC Yellow 5), 4-[(2,3-Dihydroxypropyl)-amino]-3-nitro-1-trifluormethyl-benzol (HC Yellow 6), 2-[Di-(2-hydroxyethyl)amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol Hydrochlorid (HC Yellow 9), 1-Chlor-2,4-bis[(2-hydroxyethyl)amino]-5-nitrobenzol (HC Yellow 10), 2-[(2-Hydroxyethyl)-amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethylbenzol (HC Yellow 13), 4-[(2-Hydroxyethyl)-amino]-3-nitrobenzonitril (HC Yellow 14), 4-[(2-Hydroxyethyl)amino]-3-nitrobenzamid (HC Yellow 15), 3-[(2-Hydroxyethyl)amino]-4-methyl-1-nitrobenzol, 4-Chlor-3-[(2-hydroxyethyl)amino]-1-nitrobenzol.
Bevorzugte direktziehende Nitrofarbstoffe sind die unter den internationalen Bezeichnungen bzw. Handelsnamen HC Yellow 2, HC Yellow 4, HC Yellow 5, HC Yellow 6, HC Yellow 12, HC Orange 1, HC Red 1, HC Red 3, HC Red 10, HC Red 11, HC Red 13, HC Red BN, HC Blue 2, HC Blue 11, HC Blue 12, HC Violet 1, Disperse Violet 4, bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxy-ethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Als anionische direktziehende Farbstoffe eignen sich insbesondere 6-Hydroxy-5-[(4-sulfophenyl)-azo]-2-naphthalinsulfonsäure Dinatriumsalz (C.I. 15,985; Food Yellow No. 3; FD&C Yellow No. 6), 2,4-Dinitro-1-naphthol-7-sulfonsäure Dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure Dinatriumsalz (C.I. 13,015, Acid Yellow 9), 5-Hydroxy-1-(4-sulfophenyl)-4-[(4-sulfophenyl)azo]pyrazol-3-carbonsäure Trinatriumsalz (C.I. 19,140; Food Yellow No. 4; Acid Yellow 23), 3-[(4-Phenylamino)phenyl]azobenzolsulfonsäure Natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylaminobenzolsulfonsäure Natriumsalz (C.I. 10,385; Acid Orange 3), 4-[(2,4-Dihydroxyphenyl)azo]benzolsulfonsäure Natriumsalz (C.I. 14,270; Acid Orange 6), 4-[(2-Hydroxynaphth-1-yl)azo]benzolsulfonsäure Natriumsalz (C.I. 15,510;
Acid Orange 7), 4-[(2,4-Dihydroxy-3-[(2,4-dimethylphenyl)azo]-phenyl)azo]benzolsulfonsäure Natriumsalz (C.I. 20,170; Acid Orange 24), 4-Hydroxy-3-[(2-methoxyphenyl)azo]-1-naphthalinsulfonsäure Natriumsalz (C.I. 14,710; Acid Red 4), 4-Hydroxy-3-[(4-sulfonaphth-1-yl)azo]-1-naphthalinsulfonsäure Dinatriumsalz (C.I. 14,720; Acid Red No. 14), 6- Hydroxy-5-[(4-sulfonaphth-1-yl)azo]-2,4-naphthalindisulfonsäure Trinatriumsalz (C.I. 16,255; Ponceau 4R; Acid Red 18), 3-Hydroxy-4-[(4-sulfonaphth-1-yl)azo]-2,7-naphthalindisulfonsäure Trinatriumsalz (C.I. 16,185; Acid Red 27), 8-Amino-1-hydroxy-2-(phenylazo)-3,6-naphthalindisulfonsäure Dinatriumsalz (C.I. 17,200; Acid Red 33; Red 33), 5-(Acetylamino)-4-hydroxy-3-[(2-methylphenyl)azo]-2,7-naphthalindisulfonsäure Dinatriumsalz (C.I. 18,065; Acid Red 35), 2-(3-Hydroxy-2,4,5,7-tetraiod-dibenzopyran-6-on-9-yl)benzoesäure Dinatriumsalz (C.I. 45,430; Acid Red 51), N-[6-(Diethylamino)-9-(2,4-disulfophenyl)-3H-xanthen-3-yliden]-N-ethylethanammonium hydroxid, inneres Salz, Natriumsalz (C.I. 45,100; Acid Red 52), 8-[(4-(Phenylazo)phenyl)azo]-7-naphthol-1,3-disulfonsäure Dinatriumsalz (C.I. 27,290; Acid Red 73), 2',4',5',7'-Tetrabrom-3',6'-dihydroxyspiro[isobenzofuran-1(3H),9'-[9H]xanthen]-3-on Dinatriumsalz (C.I. 45,380; Acid Red 87), 2',4',5',7'-Tetrabrom-4,5,6,7-tetrachlor-3',6'-dihydroxyspiro[isobenzofuran-1(3H),9'[9H]xanthen]-3-on Dinatriumsalz (C.I. 45,410; Acid Red 92), 3',6'-Dihydroxy-4',5'-diiodospiro[isobenzofuran-1(3H),9'(9H)-xanthen]-3-on Dinatriumsalz (C.I. 45425; Acid Red 95), 2-Hydroxy-3-[(2-hydroxynaphth-1-yl)azo]-5-nitrobenzolsulfonsäure Natriumsalz (C.I. 15,685; Acid Red 184), 3-Hydroxy-4-(3-methyl-5-oxo-1-phenyl-4,5-dihydro-1H-pyrazol-4-ylazo)naphthalin-1-sulfonsäure Natriumsalz, Chrom-Komplex (Acid Red 195), 3-Hydroxy-4-[(4-methyl-2-sulfonphenyl)azo]-2-naphthalincarbonsäure Calciumsalz (C.I. 15,850:1; Pigment Red 57:1), 3-[(2,4-Dimethyl-5-sulfophenyl)azo]-4-hydroxy-1-naphthalinsulfonsäure Dinatriumsalz (C.I. 14,700; Food Red No. 1; Ponceau SX; FD&C Red No. 4), 1,4-Bis-(2-sulfo-4-methylphenyl)amino]-9,10-anthrachinon Dinatriumsalz (C.I. 61,570; Acid Green 25), Bis-[4-(dimethylamino)phenyl]-(3,7-disulfo-2-hydroxynaphth-1-yl)carbenium-inneres Salz, Natriumsalz (C.I. 44,090; Food Green No. 4; Acid Green 50), Bis[4-(diethylamino)phenyl]-(2,4-disulfophenyl)carbenium inneres Salz, Natriumsalz (2:1) (C.I. 42,045; Food Blue No. 3; Acid Blue 1), Bis-[4-(diethylamino)phenyl]-(5-hydroxy-2,4-disulfophenyl)carbenium, inneres Salz, Calciumsalz (2:1) (C.I. 42,051; Acid Blue 3), N-[4-[(2,4-Disulfophenyl)[4-[ethyl(phenylmethyl)amino)phenyl]methylen]-2,5-cyclohexadien-1-yliden]-N-ethylbenzolmethan-aminium hydroxid, inneres Salz, Natriumsalz (C.I. 42,080; Acid Blue 7), (2-Sulfophenyl)di[4-(ethyl((4-sulfophenyl)methyl)amino)phenyl]carbenium Dinatriumsalz Betain (C.I. 42,090; Acid Blue 9; FD&C Blue No. 1), 1-Amino-4-(phenylamino)-9,10-anthrachinon-2-sulfonsäure (C.I. 62,055; Acid Blue 25), 1-Amino-4-(cyclohexylamino)-9,10-anthrachinon-2-sulfonsäure Natriumsalz (C.I. 62045; Acid Blue 62), 2-(1,3-Dihydro-3-oxo-5-sulfo-2H-indol-2-yliden)-2,3-dihydro-3-oxo-1H-indol-5-sulfonsäure Dinatriumsalz (C.I. 73,015; Acid Blue 74), 9-(2-Carboxyphenyl)-3-[(2-methylphenyl)amino]-6-[(2-methyl-4-sulfophenyl)amino]xanthylium, inneres Salz, Natriumsalz (C.I. 45,190; Acid Violet 9), 1-Hydroxy-4-[(4-methyl-2-sulfophenyl)amino]-9,10-anthrachinon Natriumsalz (C.I. 60,730; D&C Violett No. 2; Acid Violet 43), Bis-[3-nitro-4-[(4-phenylamino)-3-sulfophenylamino]phenyl]sulfon (C.I. 10,410; Acid Brown 13), 5-Amino-4-hydroxy-6-[(4-nitrophenyl)azo]-3-(phenylazo)-2,7-naphthalindisulfonsäure Dinatriumsalz (C.I. 20,470; Acid Black 1), 3-Hydroxy-4-[(2-hydroxynaphth-1-yl)azo]-7-nitro-1-naphthalinsulfonsäure chromkomplex (3:2) (C.I. 15,711; Acid Black 52), 4-(Acetylamino)-5-hydroxy-6-[(7-sulfo-4-[(4-sulfophenyl)azo]naphth-1-yl)azo]-1,7-naphthalindisulfonsäure Tetranatriumsalz (C.I. 28,440; Food Black No. 1), 3',3",5',5"-Tetrabromphenolsulfonphthalein (Bromphenolblau), 3,3',3",4,5,5',5",6-Octabromphenolsulfonphthalein (Tetrabromphenolblau).

Bevorzugte anionische direktziehende Farbstoffe sind die unter den internationalen Bezeichnungen oder Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Acid Red 92 und Tetrabromphenolblau bekannten Verbindungen.

Als kationische direktziehende Farbstoffe eignen sich insbesondere 9-(Dimethylamino)benzo-[a]phenoxazin-7-ium Chlorid (C.I. 51,175; Basic Blue 6), Di-[4-(diethylamino)phenyl][4-(ethylamino)-naphthyl]carbenium Chlorid (C.I. 42,595; Basic Blue 7), Di-(4-dimethylaminophenyl)-[4-(methylphenylamino)naphthalin-1-yl]carbenium Chlorid (C.I. 42,563; Basic Blue 8), 3,7-Di-(dimethyl-amino)phenothiazin-5-ium Chlorid (C.I. 52,015 Basic Blue 9), Di-[4-(dimethylamino)phenyl][4-(phenylamino)naphthyl]carbenium Chlorid (C.I. 44,045; Basic Blue 26), 2-[(4-(Ethyl(2-hydroxyethyl)-amino)phenyl)azo]-6-methoxy-3-methyl-benzothiazolium Methylsulfat (C.I. 11,154; Basic Blue 41), 8-Amino-2-brom-5-hydroxy-4-imino-6-[(3-(trimethylammonio)phenyl)amino]-1 (4H)-naphthalinon Chlorid (C.I. 56,059; Basic Blue No. 99), Bis-[4-(dimethylamino)phenyl]-[4-(methylamino)-phenyl]carbenium Chlorid (C.I. 42,535; Basic Violet 1), Tri-(4-amino-3-methylphenyl)carbenium Chlorid (C.I. 42,520; Basic Violet 2), Tri-[4-(dimethylamino)-phenyl]carbenium Chlorid (C.I. 42,555; Basic Violet 3), 2-[3,6-(Diethylamino)dibenzopyranium-9-yl]benzoesäure Chlorid (C.I. 45,170; Basic Violet 10), Di-(4-aminophenyl)(4-amino-3-methylphenyl)carbenium Chlorid (C.I. 42,510 Basic Violet 14), 1,3-Bis-[(2,4-diamino-5-methylphenyl)azo]-3-methylbenzol (C.I. 21,010; Basic Brown 4), 1-[(4-Aminophenyl)azo]-7-trimethylammonio-2-naphthol Chlorid (C.I. 12,250; Basic Brown 16), 1-[(4-Amino-2-nitrophenyl)azo]-7-trimethylammonio-2-naphthol chlorid, 1-[(4-Amino-3-nitrophenyl)azo]-7-trimethylammonio-2-naphthol Chlorid (C.I. 12,251; Basic Brown 17), 3-[(4-Amino-2,5-dimethoxyphenyl)azo]-N,N,N-trimethylbenzolaminium Chlorid (C.I. 12,605, Basic Orange 69), 3,7-Diamino-2,8-dimethyl-5-phenylphenazinium Chlorid (C.I. 50,240; Basic Red 2), 1,4-Dimethyl-5-[(4-dimethylaminophenyl)azo]-1,2,4-triazolium Chlorid (C.I. 11,055; Basic Red 22), 2-Hydroxy-1-[(2-methoxyphenyl)azo]-7-trimethylammonionaphthalin Chlorid (C.I. 12,245; Basic Red 76), Di-[4-(dimethylamino)phenyl]iminomethan Chlorid (C.I. 41,000; Basic Yellow 2), 2-[2-((2,4-Dimethoxyphenyl)amino)ethenyl]-1,3,3-trimethyl-3H-indol-1-ium Chlorid (C.I. 48,055; Basic Yellow 11), 3-Methyl-1-phenyl-4-[(3-(trimethylammonio)phenyl)azo]pyrazol-5-on Chlorid (C.I. 12,719; Basic Yellow 57), Bis-(4-diethylaminophenyl)phenylcarbenium Hydrogensulfat (1:1) (C.I. 42,040; Basic Green 1), Di-(4-dimethylaminophenyl)phenylmethanol (C.I. 42,000; Basic Green 4), 1-(2-Morpholiniumpropylamino)-4-hydroxy-9,10-anthrachinon Methylsulfat, 1-[(3-(Di-methylpropylamminium)propyl)amino]-4-methylamino-9,10-anthrachinon Chlorid und direktziehende Farbstoffe, die einen Heterocyclus enthalten, der mindestens ein quaternäres Stickstoffatom aufweist.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe.

Die kationischen direktziehenden Farbstoffe, die unter dem Warenzeichen Arianor vertrieben werden, sind erfindungsgemäß ebenfalls ganz besonders bevorzugte Farbstoffe.

Weitere, einsetzbare direktziehende Farbstoffe sind Chinonfarbstoffe und neutrale Azofarbstoffe. Geeignete Chinonfarbstoffe sind insbesondere: 1,4-Di-[(2,3-dihydroxypropyl)amino]-9,10-anthrachinon, 1,4-Di-[(2-hydroxyethyl)amino]-9,10-anthrachinon (Disperse Blue 23), 1-[(2-Hydroxyethyl)-amino]-4-methylamino-9,10-anthrachinon (Disperse Blue 3), 2-[(2-Aminoethyl)amino]-9,10-anthrachinon (HC Orange 5), 1-Amino-4-hydroxy-9,10-anthrachinon (Disperse Red 15), 1-Hydroxy-4-[(4-methyl-2-sulfophenyl)amino]-9,10-anthrachinon, 7-β-D-Glucopyranosyl-9,10-dihydro-1-methyl-9,10-dioxo-3,5,6,8-tetrahydroxy-2-anthracencarbonsäure (Natural Red 4), 1-[(3-Aminopropyl)amino]-4-methylamino-9,10-anthrachinon (HC Blue 8), 1-[(3-Aminopropyl)-amino]-9,10-anthrachinon (HC Red 8), 1,4-Diamino-2-methoxy-9,10-anthrachinon (Disperse Red 11, Solvent Violet No. 26), 1,4-Dihydroxy-5,8-bis[(2-hydroxyethyl)amino]-9,10-anthrachinon (Disperse Blue 7, Solvent Blue No. 69), 1,4-Diamino-9,10-anthrachinon (C.I. 61,100, Disperse Violet 1), 1-Amino-4-(methylamino)-9,10-anthrachinon (Disperse Violet 4, Solvent Violet No. 12), 2-Hydroxy-3-methoxy-1,4-naphthochinon, 2,5-Dihydroxy-1,4-naphthochinon, 2-Hydroxy-3-methyl-1,4-naphthochinon, N-{6-[(3-Chlor-4-(methylamino)phenyl)imino]-4-methyl-3-oxo-1,4-cyclohexadien-1-yl}harnstoff (HC Red 9), 2-{{4-[Di-(2-hydroxyethyl)amino]phenyl}amino}-5-[(2-hydroxyethyl)amino]-2,5-cyclohexadien-1,4-dion (HC Green 1), 5-Hydroxy-1,4-naphthochinon (Natural Brown 7), 2-Hydroxy-1,4-naphthochinon (Natural Orange 6), 1,2-Dihydro-2-(1,3-dihydro-3-oxo-2H-indol-2-yliden)-3H-indol-3-on (C.I. 73,000), 4-{{5-[(2-Hydroxyethyl)amino]-1-methyl-1H-pyrazol-4-yl}imino}-4,5-dihydro-5-[(2-hydroxyethyl)imino]-1-methyl-1 H-pyrazol Sulfat (1:1), Hydrat(1:1). Geeignete neutrale Azofarbstoffe sind insbesondere: 1-[Di-(2-hydroxyethyl)amino]-3-methyl-4-[(4-nitrophenyl)azo]benzol (Disperse Red 17), 1-[Di-(2-hydroxyethyl)amino]-4-[(4-nitrophenyl)azo]benzol (Disperse Black 9), 4-[(4-Aminophenyl)azo]-1-[di-(2-hydroxyethyl)amino]-3-methylbenzol (HC Yellow 7), 2,6-Diamino-3-[(pyridin-3-yl)azo]pyridin, 2-{[4-(Acetylamino)phenyl]azo}-4-methylphenol (Disperse Yellow 3), 4-[(4-Nitrophenyl)azo]anilin (Disperse Orange 3).

Eine weitere Ausführungsform der vorliegenden Erfindung ist dadurch gegeben, dass das Mittel mindestens einen direktziehenden Farbstoff enthält, der ausgewählt ist aus der Gruppe, die ist gebildet aus Acid Red 92, Tetrabromphenolblau und HC Yellow No.13.

Dabei kann eine weitere Ausführungsform der vorliegenden Erfindung dadurch gekennzeichnet sein, dass das Mittel mindestens 2 direktziehende Farbstoffe enthält. Bevorzugt enthalten solche Mittel dabei mindestens Acid Red 92 und Tetrabromphenolblau oder mindestens Tetrabromphenolblau und HC Yellow No.13 oder Acid Red 92 und Tetrabromphenolblau und HC Yellow No.13 sowie gegebenenfalls weitere direktziehende Farbstoffe.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Die erfindungsgemäßen Mittel sind oxidative Haarfärbemittel

Als Oxidationsmittel wird Wasserstoffperoxid eingesetzt. Bevorzugt beträgt die Menge an Wasserstoffperoxid im anwendungsbereiten Mittel 0,5 bis 12 Gew.-%, bevorzugt 1 bis 8 Gew.-%, jeweils bezogen auf das anwendungsbereite Mittel.

Die vorliegenden Mittel, sind dadurch gekennzeichnet ist, dass die Mittel zusätzlich als weiteres chemisches Oxidationsmittel eine feste Peroxoverbindung enthält, welche ausgewählt wird aus der Gruppe, die gebildet wird aus Ammoniumperoxodisulfat, Kaliumperoxodisulfat, Natriumperoxodisulfat, Ammoniumperoxomonosulfat, Kaliumperoxomonosulfat, Natriumperoxomonosulfat, Alkalimetallperoxodiphosphaten wie Kaliumperoxodiphosphat, Percarbonaten wie Magnesiumpercarbonat, Peroxiden wie Bariumperoxid, Perboraten wie Natriumperborat, sowie Wasserstoffperoxid-Anlagerungsverbindungen an feste Träger, wie Harnstoffperoxid und Melaminperoxid.

Bevorzugte Peroxodisulfatsalze sind Ammoniumperoxodisulfat, Kaliumperoxodisulfat und Natriumperoxodisulfat. Es kann dabei bevorzugt sein, eine Kombination aus Ammoniumperoxodisulfat und mindestens einem Alkalimetallperoxodisulfat einzusetzen. Die festen Peroxoverbindungen sind in den erfindungsgemäßen Mitteln in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Weiterhin hat es sich als vorteilhaft herausgestellt, wenn das erfindungsgemäße Mittel in Form einer fließfähigen Zubereitung vorliegt. Bevorzugt enthalten die fließfähigen Zubereitungen zusätzlich als oberflächenaktive Substanz einen Emulgator bzw. ein weiteres Tensid, wobei oberflächenaktive Substanzen je nach Anwendungsgebiet als Tenside oder als Emulgatoren bezeichnet werden und aus amphoteren, anionischen, kationischen, zwitterionischen und nichtionischen Tensiden und Emulgatoren ausgewählt sind.

In einer besonders bevorzugten Ausführungsform enthält das erfindungsgemäße Mittel weiterhin mindestens ein amphoteres Tensid. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside werden unter der INCI-Bezeichnung Disodium Cocoamphodipropionate und der Bezeichnung Disodium Cocoamphodiacetate vermarktet. Ganz besonders bevorzugte ist Disodium Cocoamphodipropionate. Der Zusatz im erfindungsgemäßen Mittel bewirkt eine Verbesserung des Farberhalts.

Als anionische Tenside eignen sich in erfindungsgemäßen Zubereitungen alle für die Verwendung am menschlichen Körper geeigneten anionischen oberflächenaktiven Stoffe. Beispiele für geeignete anionische Tenside, jeweils in Form der Natrium-, Kalium- und Ammonium- sowie der Mono, Di- und Trialkanolammoniumsalze, sind lineare und verzweigte, gesättigte und ungesättigte Fettsäuren (Seifen); gegebenenfalls polyalkoxylierte Ethercarbonsäuren der Formel RO(CH₂CH₂O)ₓCH₂COOH, in der R eine lineare Alkylgruppe und x = 0 oder eine Zahl von 1 bis 16 ist; Acylsarcoside; Acyltauride; Acylisethionate; Alkylsulfate und Alkylethersulfate der Formel RO(CH₂CH₂O)ₓSO₃H, in der R eine lineare Alkylgruppe und x = 0 oder eine Zahl von 1 bis 12 ist; Alkyl- und/oder Alkenyletherphosphate der Formel RO(C₂H₄O)ₓP(=O)(OH)OR', in der R bevorzugt für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht. Bevorzugte anionische Tenside sind Fettsäuren, Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethyl-ammoniumglycinate und 2-Alkyl-3-carboxymethyl-3-hydroxyethyl-imidazoline mit jeweils 8 bis 18 C-Atomen in der Alkyl- oder Acylgruppe. Ein bevorzugtes zwitterionisches Tensid ist das unter der INCI-Bezeichnung bekannte Cocamidopropyl Betain.

Weiterhin hat es sich als vorteilhaft erwiesen, wenn die erfindungsgemäßen Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 2 bis 50 Mol Ethylenoxid und/oder 0 bis 5 Mol Propylenoxid an lineare und verzweigte Fettalkohole mit 8 bis 30 C-Atomen; C₁₂-C₃₀-Fettsäuremono- und -diester von Anlagerungsprodukten von 1 bis 30 Mol Ethylenoxid an Glycerin; Polyglycerinester und alkoxylierte Polyglycerinester; alkoxylierte, bevorzugt propoxylierte und insbesondere ethoxylierte, Mono-, Di- und Triglyceride; Aminoxide; Sorbitanfettsäureester und Anlagerungeprodukte von Ethylenoxid an Sorbitanfettsäureester; Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21 Kohlenstoffatomen in der Alkylkette sowie Alkylpolyglykoside. Als nichtionische Tenside eignen sich insbesondere alkoxylierte, bevorzugt ethoxylierte Fettalkohole, C₈-C₂₂-Alkylmono- und -oligoglycoside und deren ethoxylierte Analoga.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, wie Alkyltrimethylammoniumchloride, Dialkyldimethylammoniu chloride und Trialkylmethylammoniumchloride, sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Ebenfalls erfindungsgemäß vorteilhaft sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Solche Produkte werden beispielsweise unter den Warenzeichen Stepantex, Dehyquart und Armocare vertrieben.

Die anionischen, nichtionischen, zwitterionischen und amphoteren Tenside werden in einer Gesamtmenge von 0,1 bis 45 Gew.%, bevorzugt 1 bis 30 Gew.% und besonders bevorzugt von 1 bis 20 Gew.%, bezogen auf die Gesamtmenge des anwendungsbereiten Mittels, eingesetzt. Die kationischen Tenside sind in den erfindungsgemäß verwendeten Mitteln bevorzugt in Mengen von 0,05 bis 10 Gew.-%, bezogen auf das gesamte Mittel, enthalten.

Es hat sich erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Bevorzugt handelt es sich bei dem Verdickungsmittel um ein anionisches, synthetisches Polymer. Bevorzugte anionische Polymere sind Acrylsäure- und/oder Methacrylsäure-Polymerisate oder - Copolymerisate, die in den erfindungsgemäßen Mitteln bevorzugt in einer Menge von 0,1 bis 10 Gew.-%, besonders bevorzugt von 0,5 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogene Monomereinheiten bevorzugt Acrylamid, Methacrylamid, Acrylsäure-C₁-C₄-Alkylester und Methacrylsäure-C₁-C₄-Alkylester eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.

Ferner können die erfindungsgemäßen Mittel weitere Wirk-, Hilfs- und Zusatzstoffe enthalten, wie beispielsweise
nichtionische Polymere, kationische Polymere, zwitterionische und amphotere Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di-und Oligosaccharide wie beispielsweise Glucose, Galactose, Fructose, Fruchtzucker und Lactose, Entschäumer wie Silikone, bevorzugt Dimethicon, Farbstoffe zum Anfärben des Mittels, Antischuppenwirkstoffe, Lichtschutzmittel, Pflegestoffe wie Aminosäuren, Oligopeptide, Proteinhydrolysate, Silikonderivate, Polyphenole und (Pseudo)Ceramide, Wirkstoffe wie Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell-und Penetrationsstoffe wie Glycerin, Propylenglykolmonoethylether, Carbonate, Hydrogencarbonate, Guanidine, Harnstoffe sowie primäre, sekundare und tertiäre Phosphate, Trübungsmittel wie Latex, Styrol/PVP- und Styrol/Acrylamid-Copolymere, Perlglanzmittel wie Ethylenglykolmono-und -distearat sowie PEG-3-distearat, Pigmente, Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Üblicherweise werden die Wirk-, Hilfs- und Zusatzstoffe in Mengen von 0,001 bis 15 Gew.-%, vorzugsweise von 0,005 bis 5 Gew.-%, jeweils bezogen auf das anwendungsbereite Mittel, eingesetzt.

Erfindungsgemäß bevorzugte Mittel zur Farbänderung sind dadurch gekennzeichnet, dass sie einen möglichst neutralen pH-Wert besitzen. Eine weitere bevorzugte Ausführungsform der vorliegenden Erfindung besteht darin, dass das anwendungsbereite Mittel einen pH-Wert zwischen 4,0 und 12,0, bevorzugt zwischen 5,0 und 11,5, insbesondere bevorzugt zwischen 6,0 und 11,0 besitzt. Bei den pH-Werten im Sinne der vorliegenden Erfindung handelt es sich um pH-Werte, die bei einer Temperatur von 22 °C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.
Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol (Monoethanolamin), Triethanolamin, Ammoniak, und 1-Aminopropan-2-ol.
Die Konfektionierung der erfindungsgemäßen Färbemittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der erfindungsgemäßen Komponenten separat verpackt wird; es kann aber bevorzugt sein, die Zubereitung, die das erfindungsgemäße Copolymer enthält, bis zur Anwendung separat von zu verpacken.
Die erfindungsgemäßen Mittel können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt.

Ein weiterer Gegenstand der vorliegenden Erfindung ist daher eine Mehrkomponentenverpackungseinheit (Kit-of-Parts), enthaltend mindestens zwei drei getrennt voneinander konfektionierte Container, wobei
- ein erster Container (I) eine Farbveränderungszubereitung (A), enthaltend in einem kosmetischen Träger als farbgebende Komponente mindestens einen direktziehenden Farbstoff, ausgewählt aus der Gruppe, gebildet aus Nitrofarbstoffen, kationischen direktziehenden Farbstoffen und anionischen direktziehenden Farbstoffen, enthält,
- ein zweiter Container (II) eine fließfähige Oxidationsmittelzusammensetzung (B), enthaltend als chemisches Oxidationsmittel mindestens insbesondere Wasserstoffperoxid, enthält, und
- ein dritter Container (III) eine Bleichkraftverstärkerzubereitung (C) enthält,
dadurch gekennzeichnet, dass
- entweder die Farbveränderungszubereitung (A) und/oder die Oxidationszubereitung (B) mindestens ein nicht permanent ionisches Copolymer enthält, welches sich von mindestens einem kationisierbaren Monomer der allgemeinen Formel (F1), 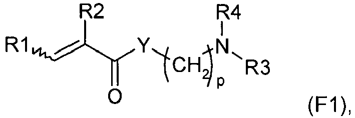 worin R1 für Wasserstoff, R2, R3 und R4 jeweils für eine Methylgruppe, Y für eine NH
- Gruppe und p für die Zahl 3 steht, und von N-Vinyl-pyrrolidin-2-on ableitet,
mit der Massgabe,
dass das nicht permanent ionische Copolymer in der Oxidationsmittelzubereitung (B) enthalten ist, wenn die Farbveränderungszubereitung (A) als farbgebende Komponente mindestens ein Oxidationsfarbstoffvorprodukt enthält, und
weiterhin dadurch gekennzeichnet, dass
- die Bleichkraftverstärkerzubereitung (C) wasserfrei ist und mindestens eine feste Peroxoverbindung enthält, die ausgewählt wird aus der Gruppe, die gebildet wird aus Ammoniumperoxodisulfat, Kaliumperoxodisulfat, Natriumperoxodisulfat, Ammoniumperoxomonosulfat, Kaliumperoxomonosulfat, Natriumperoxomonosulfat, Alkalimetallperoxodiphosphaten wie Kaliumperoxodiphosphat, Magnesiumpercarbonat, Bariumperoxid, Natriumperborat sowie Harnstoffperoxid und Melaminperoxid.

Unter Container wird im Rahmen der vorliegenden Erfindung eine Umhüllung verstanden, die in Form einer gegebenenfalls wieder-verschließbaren Flasche, einer Dose, eines Tütchens, eines Sachets oder ähnlichen Umhüllungen vorliegt. Dabei kann die Umhüllung aus Kunststoff, Glas, (Metall-) blech, Karton, Papier oder einem Verbundstoff gefertigt sein.

Die Oxidationsmittelzubereitungen (B) sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.
Die Oxidationsmittelzubereitung enthält neben dem eigentlichen Oxidationsmittel weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel wurden vorangehend ausführlich beschrieben. Sie werden bevorzugt in Anteilen von 0,05 bis 10 Gew.-%, bevorzugt von 0,1 bis 3 Gew.-%, jeweils bezogen auf die anwendungsbereiten Mittel, eingesetzt.

Weiterhin hat es sich als vorteilhaft erweisen, wenn die Oxidationsmittelzubereitungen aus oxidativen Färbemitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Bevorzugt sind die Komplexbildner im erfindungsgemäßen Mittel zu 0,05 bis 10 Gew.-%, bevorzugt zu 0,5 bis 5 Gew.-%, jeweils bezogen auf das Gesamtgewicht des anwendungsbereiten Mittels, enthalten.

Die Mehrkomponentenverpackungseinheit (Kit-of-Parts) ist dadurch gekennzeichnet, dass sie einen zusätzlichen, getrennt konfektionierten Container (III) enthält, der eine Bleichkraftverstärkerzubereitung (C) enthält.

Obwohl prinzipiell keine Einschränkungen hinsichtlich der Formulierung der Bleichkraftverstärkerzubereitung (C) bestehen, hat es sich erfindungsgemäß als bevorzugt erwiesen, wenn diese Zubereitung wasserfrei formuliert ist. In einer Ausführungsform dieses Erfindungsgegenstands ist die Bleichkraftverstärkerzubereitung (C) daher wasserfrei und enthält mindestens eine feste Peroxoverbindung. Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser enthalten, können erfindungsgemäß besonders bevorzugt sein. Die Zubereitung (C) ist vorzugsweise als gegebenenfalls entstaubtes Pulver, als wasserfreie Paste oder in Form gepressten Formkörpers formuliert.

Im Falle der Formulierung als wasserfreie Paste hat es sich als besonders bevorzugt erwiesen, wenn die Zubereitung C mindestens einen nicht hydroxylierten Fettsäureester mit einem Schmelzpunkt von höchstens 50°C, insbesondere von höchstens 30°C, und/oder mindestens eine C₁₀-C₃₀-Fettsäure mit mindestens einer zusätzlichen Hydroxygruppe und/oder ein Derivat davon, enthält. Ester von nicht hydroxylierten C₆-C₃₀-Alkylmonocarbonäuren mit C₂-C₃₀-Monoalkoholen eignen sich erfindungsgemäß bevorzugt als Fettsäureester.

Die feste Peroxoverbindung wird ausgewählt aus der Gruppe, die gebildet wird aus Ammonium-peroxodisulfat, Kaliumperoxodisulfat, Natriumperoxodisulfat, Ammoniumperoxomonosulfat, Kaliumperoxomonosulfat, Natriumperoxomonosulfat, Alkalimetallperoxodiphosphaten wie Kaliumperoxodiphosphat, Percarbonaten wie Magnesiumpercarbonat, Peroxiden wie Bariumperoxid, Perboraten wie Natriumperborat, sowie Wasserstoffperoxid-Anlagerungsverbindungen an feste Träger, wie Harnstoffperoxid und Melaminperoxid.

Im Zusammenhang der Untersuchungen zur erfindungsgemäßen Mehrkomponentenverpackungseinheit hat es sich herausgestellt, dass das erfindungsgemäße, nicht permanent ionische Copolymer gemäß Formel (F1) zur Verbesserung des Farberhalts sowohl in der Farbveränderungszubereitung (A) als auch in der Oxidationszubereitung (B) enthalten sein kann.

Eine besondere Ausführungsform dieses Erfindungsgegenstands ist daher dadurch gekennzeichnet, dass das nicht permanent ionische Copolymer in der Farbveränderungszubereitung (A) enthalten ist, mit der Massgabe, dass die Farbveränderungszubereitung (A) als farbgebende Komponente kein Oxidationsfarbstoffvorprodukt enthält. Eine andere Ausführungsform dieses Erfindungsgegenstands ist daher dadurch gekennzeichnet, dadurch gekennzeichnet, dass das nicht permanent ionische Copolymer in der Oxidationszubereitung (B) enthalten ist.
Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Applikationshilfe, wie beispielsweise ein Kamm oder ein Pinsel, und/oder eine persönliche Schutzausrüstung, wie beispielsweise Einweg-Handschuhe dem Kit beigefügt ist. Bevorzugt werden die Zubereitungen (A) und (B) sowie gegebenenfalls (C) der Mehrkomponentenverpackungseinheit dieses Erfindungsgegenstands miteinander zum anwendungsbereiten Färbemittel vermischt. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.
Ein weiterer Gegenstand der vorliegenden Erfindung ist ein Verfahren, bei dem ein erfindungsgemäßes Mittel gemäß obiger Vorgaben auf keratinische Fasern, insbesondere menschliches Haar, aufgetragen wird, für eine Einwirkzeit von 2 bis 60 Minuten, bevorzugt von 5 bis 45 Minuten auf dem Haar belassen wird, und anschließend das Haar ausgespült wird. Die Anwendungstemperaturen bei der erfindungsgemäßen Farbveränderung keratinischer Fasern können in einem Bereich zwischen 15 und 45 °C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Eine weitere Ausführungsform dieses Erfindungsgegenstands ist ein Verfahren zum Färben menschlicher Haare, das dadurch gekennzeichnet ist, dass die Zubereitungen (A), (B) sowie (C) der getrennt konfektionierten Container (I), (II) sowie (III) einer Mehrkomponentenverpackungseinheit nach dem zweiten Erfindungsgegenstand innig miteinander vermischt werden, die resultierende Anwendungszubereitung auf das Haar aufgetragen wird, für eine Einwirkzeit von 2 bis 60 Minuten, bevorzugt von 5 bis 45 Minuten auf dem Haar belassen wird, und anschließend das Haar ausgespült wird.

Bezüglich weiterer bevorzugter Ausführungsformen der erfindungsgemäßen Verfahren gilt mutatis mutandis das zu den erfindungsgemäßen Mitteln Gesagte.

Ein weiterer Gegenstand der Erfindung ist die Verwendung der Mittel des ersten Erfindungsgegenstandes zur Verbesserung des Farberhalts, insbesondere gegenüber Haarwäsche, nach Farbveränderung menschlicher Haare.

Ein weiterer Gegenstand der vorliegenden Erfindung ist die Verwendung eines nach Vermischung der Komponenten einer Mehrkomponentenverpackungseinheit des zweiten Erfindungsgegenstands hergestellten, anwendungsbereiten Mittels zur Verbesserung des Farberhalts bei der Färbung keratinischer Fasern, insbesondere menschlicher Haare.

Bezüglich weiterer bevorzugter Ausführungsformen der erfindungsgemäßen Verwendungen gilt mutatis mutandis das zu den erfindungsgemäßen Mitteln Gesagte.

Die nachfolgenden Beispiele veranschaulichen erfindungsgemäße Ausführungsformen und sind keinesfalls einschränkend für die vorliegende Erfindung zu verstehen.

### Beispiele

Folgende Färbecremes wurden hergestellt:

| **Rohstoffe** | **E1** | **E2** |
|---|---|---|
| Tetrabromphenolblau | 0,12 | 0,12 |
| D&C Red No. 28 | 0,04 | 0,04 |
| 4-Amino-3-methylphenol | --- | --- |
| p-Toluylendiaminsulfat | --- | --- |
| 2-Amino-3-hydroxypyridin | --- | --- |
| 5-Amino-2-methylphenol | --- | --- |
| 1-Naphthol | --- | --- |
| Resorcin | --- | --- |
| 4-Amino-6-chloro-4-nitrophenol | --- | --- |
| Aerosil 200 V | --- | --- |
| Ammonium Carbomer, 1% | 15,00 | 14,94 |
| Lanette E, Pulver | 0,70 | 0,70 |
| Texapon NSF, 27% | 4,40 | 4,38 |
| Kalium Oleat, 12,5% | 3,00 | 2,99 |
| Cutina GMS SE | 2,00 | 1,99 |
| Cutina AGS | 2,00 | 1,99 |
| Eutanol G | 2,00 | 1,99 |
| Hydrenol D | 12,00 | 11,95 |
| Eumulgin B2 | 3,00 | 2,99 |
| Titandioxid | 0,50 | 0,50 |
| Phospholipid EFA | 0,10 | 0,10 |
| Na₄-EDTA, Pulver, 87% | 0,20 | 0,20 |
| Merquat Plus 3330 | 1,50 | 1,49 |
| Styleze CC10 | 1,00 | 1,00 |
| Citronensäure | 0,50 | 0,50 |
| Ascorbinsärue | --- | --- |
| HEDP, 60% | --- | 0,40 |
| Natriumsulfit, 96% | --- | --- |
| Natriumchlorid | --- | 0,40 |
| Phenoxyethanol | 1,00 | --- |
| Crodarom Rock Crystal | 0,10 | 0,10 |
| Puricare LS9658 | 1,00 | 1,00 |
| Ammoniak, 25% | 2,00 | 2,00 |
| Kaliumhydroxid, 50% | --- | --- |
| Parfum | qs | qs |
| Wasser | ad 100 | ad 100 |

- Aerosil 200 V: Silciumdioxid, pyrogen (INCI-Bezeichnung: Silica) (Evonik)
- Lanette E, Pulver: C₁₆-C₁₈-Fettalkoholsulfat, Natrium-Salz (INCI-Bezeichnung: Sodium Cetearyl Sulfate) (Cognis)
- Texapon NSF, 27%: C₁₂-Fettalkoholsulfat, ethoxyliert (2 EO) Natrium-Salz (INCI-Bezeichnung: Sodium Laureth Sulfate) (Cognis)
- Cutina GMS SE: Stearinsäureglycerylester (INCI-Bezeichnung: Glyceryl Stearate) (Cognis)
- Cutina AGS: Ethylenglycoldistearylester (INCI-Bezeichnung: Glycol Distearate) (Cognis)
- Eutanol G: 2-Octyldodecanol (INCI-Bezeichnung: Octyldodecanol) (Cognis)
- Hydrenol D: C₁₆-C₁₈-Fettalkohol (INCI-Bezeichnung: Cetearyl alcohol) (Cognis)
- Eumulgin B2: C₁₆-C₁₈-Fettalkohol, ethoxyliert (20 EO) (INCI-Bezeichnung: Ceteareth-20) (Cognis)
- Phospholipid EFA: zwitterionisches Phospholid (INCI-Bezeichnung: Linoleamidopropyl PG-dimonium chloride phosphate) (Uniqema)
- Merquat Plus 3330: Terpolymer aus Acrylsäure, Acrylamid und Dimethyldiallylammonium Chlorid (ca. 10% Aktivsubstanz; INCI-Bezeichnung: Polyquaternium-39) (Nalco)
- Styleze CC10: Copolymer aus Vinylpyrrolidinon u. Dimethylaminopropylmethacrylat (ca. 10 % Aktivsubstanz; INCI-Bezeichnung: VP/DMAPA Acrylates Copolymer) (ISP)
- Crodarom Rock Crystal: Quartzextrakt (INCI-Bezeichnung: Aqua, Propylene Glycol, Quartz) (Crodarom)
- Puricare LS9658: Moringa Oleifera-Samen-Extrakt (INCI-Bezeichnung: Aqua, Glycerine, Moringa Pterygosperma Seed Extract) (Cognis France)
Blondierpulver C:

| **Rohstoff** | **Menge [Gew.-%]** |
|---|---|
| Natriummetasilikat | 4,6 |
| Portil N | 38,0 |
| Rohagit S hv | 0,5 |
| Cekol 50000 | 2,5 |
| Magnesiumcarbonat | 14,2 |
| Na₂EDTA | 1,0 |
| Gluadin AGP | 0,1 |
| Kieselsäure, pyrogen | 0,5 |
| Kaliumperoxodisulfat | 20,4 |
| Natriumperoxodisulfat | 10,2 |
| Paraffinum Liquidum | 8,0 |

- Portil N: Natriumsilicat (INCI-Bezeichnung: Sodium Silicate) (Cognis)
- Rohagit S hv: Copolymer aus Methacrylsäuremethylester und Methacrylsäure (INCI-Bezeichnung: Acrylates Copolymer) (Evonik)
- Cekol 50000: Carboxymethylcellulose Natriumsalz (INCI-Bezeichnung: Cellulose Gum) (Kelco)
- Gluadin AGP: Weizenproteinhydrolysat (INCI-Bezeichnung: hydrolyzed wheat protein) (Cognis)

a) Die Färbecremes E1 und E2 wurden vor der Anwendung mit einer 6 Gew.-%igen Wasserstoffperoxid-Lösung sowie einem Blondierpulver C jeweils im Gewichtsverhältnis 3,5 Teile Creme, 5 Teile Entwickler und 1,5 Teile Blondierpulver vermischt, die anwendungsbereite Mischung wurde auf das Haar aufgebracht und nach 45 min Einwirkzeit wurde das Haar mit lauwarmen Wasser gespült.

## Patentansprüche

1. Mittel zur Farbveränderung von keratinischen Fasern, insbesondere menschlichen Haaren, enthaltend in einem kosmetischen Träger mindestens einen direktziehenden Farbstoff, ausgewählt aus der Gruppe, gebildet aus Nitrofarbstoffen, kationischen direktziehenden Farbstoffen und anionischen direktziehenden Farbstoffen,
**dadurch gekennzeichnet, dass**
- das Mittel zusätzlich ein nicht permanent ionisches Copolymer enthält, welches sich von mindestens einem kationisierbaren Monomer der allgemeinen Formel (F1), 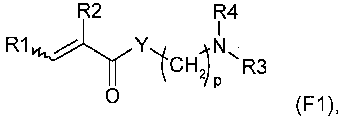 worin R1 für Wasserstoff, R2, R3 und R4 jeweils für eine Methylgruppe, Y für eine NH-Gruppe und p für die Zahl 3 steht, und von N-Vinyl-pyrrolidin-2-on ableitet und zusätzlich
- Wasserstoffperoxid und
- als weiteres chemisches Oxidationsmittel eine feste Peroxoverbindung enthält, welche ausgewählt wird aus der Gruppe, die gebildet wird aus Ammoniumperoxodisulfat, Kaliumperoxodisulfat, Natriumperoxodisulfat, Ammoniumperoxomonosulfat, Kaliumperoxomonosulfat, Natriumperoxomonosulfat, Kaliumperoxodiphosphat, Magnesiumpercarbonat, Bariumperoxid, Natriumperborat sowie Harnstoffperoxid und Melaminperoxid.

2. Mittel nach Anspruch 1, **dadurch gekennzeichnet, dass** der direktziehenden Farbstoff ausgewählt ist aus der Gruppe, gebildet aus Acid Red 92, Tetrabromphenolblau und HC Yellow No.13.

3. Mehrkomponentenverpackungseinheit (Kit-of-Parts), enthaltend mindestens drei getrennt voneinander konfektionierte Container, wobei
- ein erster Container (I) eine Farbveränderungszubereitung (A), enthaltend in einem kosmetischen Träger als farbgebende Komponente mindestens einen direktziehenden Farbstoff, ausgewählt aus der Gruppe, gebildet aus Nitrofarbstoffen, kationischen direktziehenden Farbstoffen und anionischen direktziehenden Farbstoffen, enthält,
- ein zweiter Container (II) eine fließfähige Oxidationsmittelzusammensetzung (B), enthaltend als chemisches Oxidationsmittel mindestens Wasserstoffperoxid, enthält, und
- ein dritter Container (III) eine Bleichkraftverstärkerzubereitung (C) enthält,
**dadurch gekennzeichnet, dass**
- entweder die Farbveränderungszubereitung (A) und/oder die Oxidationszubereitung (B) mindestens ein nicht permanent ionisches Copolymer enthält, welches sich von mindestens einem kationisierbaren Monomer der allgemeinen Formel (F1), 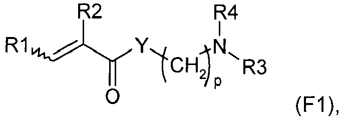 worin R1 für Wasserstoff, R2, R3 und R4 jeweils für eine Methylgruppe, Y für eine NH
- Gruppe und p für die Zahl 3 steht, und von N-Vinyl-pyrrolidin-2-on ableitet,
mit der Massgabe,
dass das nicht permanent ionische Copolymer in der Oxidationsmittelzubereitung (B) enthalten ist, wenn die Farbveränderungszubereitung (A) als farbgebende Komponente mindestens ein Oxidationsfarbstoffvorprodukt enthält, und
weiterhin **dadurch gekennzeichnet, dass**
- die Bleichkraftverstärkerzubereitung (C) wasserfrei ist und mindestens eine feste Peroxoverbindung enthält, die ausgewählt wird aus der Gruppe, die gebildet wird aus Ammoniumperoxodisulfat, Kaliumperoxodisulfat, Natriumperoxodisulfat, Ammoniumperoxomonosulfat Kaliumperoxomonosulfat, Natriumperoxomonosulfat, Alkalimetallperoxodiphosphaten wie Kaliumperoxodiphosphat, Magnesiumpercarbonat, Bariumperoxid, Natriumperborat sowie Harnstoffperoxid und Melaminperoxid.

4. Kit-of-Parts nach Anspruch 3, **dadurch gekennzeichnet, dass** das nicht permanent ionische Copolymer in der Farbveränderungszubereitung (A) enthalten ist, mit der Massgabe, dass die Farbveränderungszubereitung (A) als farbgebende Komponente kein Oxidationsfarbstoffvorprodukt enthält.

5. Kit-of-Parts nach Anspruch 3, **dadurch gekennzeichnet, dass** das nicht permanent ionische Copolymer in der Oxidationszubereitung (B) enthalten ist.

6. Verfahren zum Färben menschlicher Haare, **dadurch gekennzeichnet, dass** die Zubereitungen (A), (B) sowie (C) der getrennt konfektionierten Container (I), (II) sowie (III) einer Mehrkomponentenverpackungseinheit nach einem der Ansprüche 3 bis 5 innig miteinander vermischt werden, die resultierende Anwendungszubereitung auf das Haar aufgetragen wird, für eine Einwirkzeit von 2 bis 60 Minuten, bevorzugt von 5 bis 45 Minuten auf dem Haar belassen wird, und anschließend das Haar ausgespült wird.

## Claims

1. An agent for changing the color of keratinous fibers, in particular human hair, containing, in a cosmetic carrier, at least one direct dye selected from the group consisting of nitro dyes, cationic direct dyes and anionic direct dyes, **characterized in that**
- the agent additionally contains a non-permanent ionic copolymer that is derived from at least one cationizable monomer of general formula (F1), 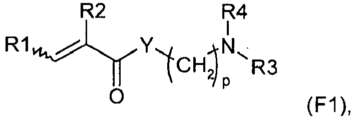 where R1 represents hydrogen, R2, R3 and R4 each represent a methyl group, Y represents an NH group and p represents the number 3, and derived from N-vinylpyrrolidin-2-one, and also contains
- hydrogen peroxide and
- a solid peroxo compound as an additional chemical oxidizing agent that is selected from the group consisting of ammonium peroxodisulfate, potassium peroxodisulfate, sodium peroxodisulfate, ammonium peroxomonosulfate, potassium peroxomonosulfate, sodium peroxomonosulfate, magnesium percarbonate, barium peroxide, sodium perborate, urea peroxide and melamine peroxide.

2. The agent according to claim 1, **characterized in that** the direct dye is selected from the group consisting of Acid Red 92, tetrabromophenol blue and HC Yellow no. 13.

3. A kit of parts, containing at least three separately packaged containers, in which
- a first container (I) contains a color-changing preparation (A), containing, in a cosmetic carrier, at least one direct dye as a color-providing component that is selected from the group consisting of nitro dyes, cationic direct dyes and anionic direct dyes,
- a second container (II) contains a flowable oxidizing agent composition (B), containing at least hydrogen peroxide as a chemical oxidizing agent, and
- a third container (III) contains a bleach enhancer preparation (C),
**characterized in that**
- either the color-changing preparation (A) and/or the oxidizing preparation (B) contains at least one non-permanent ionic copolymer that is derived from at least one cationizable monomer of general formula (F1), 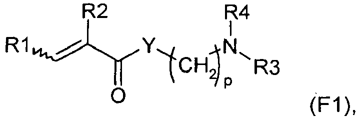 where R1 represents hydrogen, R2, R3 and R4 each represent a methyl group, Y represents an NH group and p represents the number 3, and derived from N-vinylpyrrolidin-2-one, provided that
the non-permanent ionic copolymer is contained in the oxidizing agent preparation (B) when the color-changing preparation (A) contains at least one oxidation dye precursor, and
further **characterized in that**
- the bleach enhancer preparation (C) is water-free and contains at least one solid peroxo compound that is selected from the group consisting of ammonium peroxodisulfate, potassium peroxodisulfate, sodium peroxodisulfate, ammonium peroxomonosulfate, potassium peroxomonosulfate, sodium peroxomonosulfate, alkali metal peroxodiphosphates such as potassium peroxodiphosphate, magnesium percarbonate, barium peroxide, sodium perborate, urea peroxide and melamine peroxide.

4. The kit of parts according to claim 3, **characterized in that** the non-permanent ionic copolymer is contained in the color-changing preparation (A), provided that the color-changing preparation (A) does not contain an oxidation dye precursor as a color-providing component.

5. The kit of parts according to claim 3, **characterized in that** the non-permanent ionic copolymer is contained in the oxidizing preparation (B).

6. A method for coloring human hair, **characterized in that** the preparations (A), (B) and (C) in the separately packaged containers (I), (II) and (III) in a kit of parts according to one of claims 3 to 5 are intimately mixed with one another, the resulting application preparation is applied to the hair, is left on the hair for a reaction time of from 2 to 60 minutes, preferably from 5 to 45 minutes, and is subsequently rinsed out of the hair.

## Revendications

1. Produit de coloration de fibres de kératine, en particulier de cheveux humains, contenant dans un vecteur cosmétique au moins un colorant direct choisi dans le groupe constitué des colorants nitro, des colorants directs cationiques et des colorants directs anioniques, **caractérisé en ce que** le produit contient en plus :
- un copolymère ionique non-permanent qui dérive d'au moins un monomère cationisable de formule générale F1 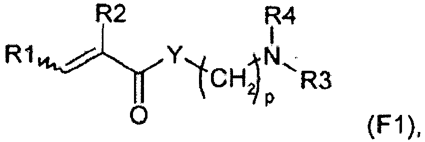 où R1 représente un hydrogène, R2, R3 et R4 représentent respectivement un groupe méthyle, Y représente un groupe NH et p = 3, et
de la N-vinyl-pyrrolidin-2-one,
- du peroxyde d'hydrogène, et
- comme autre oxydant chimique un peroxyde solide choisi dans le groupe constitué du peroxodisulfate d'ammonium, peroxodisulfate de potassium, peroxodisulfate de sodium, peroxomonosulfate d'ammonium, peroxomonosulfate de potassium, peroxomonosulfate de sodium, peroxodiphosphate de potassium, percarbonate de magnésium, peroxyde de baryum, perborate de sodium, ainsi que du peroxyde d'urée et de peroxyde de mélamine.

2. Produit selon la revendication 1, **caractérisé en ce que** le colorant direct est choisi dans le groupe constitué d'Acid Red 92, du bleu de bromophénol et de HC Yellow 13.

3. Kit multicomposant (*kit of parts*) contenant au moins trois récipients confectionnés séparément les uns des autres, où :
- un premier récipient (I) contient une préparation de coloration de cheveux (A) contenant comme composants colorants, dans un vecteur cosmétique, au moins un colorant direct choisi dans le groupe constitué des colorants nitro, des colorants directs cationiques et des colorants directs anioniques,
- un deuxième récipient (II) contient une préparation oxydante fluide (B) contenant comme oxydant chimique au moins du peroxyde d'hydrogène, et
- un troisième récipient (III) contient une préparation intensificatrice de décoloration (C),
**caractérisé en ce que** :
- la préparation de coloration de cheveux (A), et/ou la préparation oxydante (B) contient un copolymère ionique non-permanent qui dérive d'au moins un monomère cationisable de formule générale F1 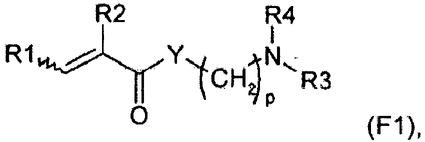 où R1 représente un hydrogène, R2, R3 et R4 représentent respectivement un groupe méthyle, Y représente un groupe NH et p = 3, et de la N-vinyl-pyrrolidin-2-one, à condition que le copolymère ionique non-permanent soit contenu dans la préparation oxydante (B) si la préparation de coloration de cheveux (A) contient comme composant colorant au moins un précurseur de colorant d'oxydation, et
**caractérisé en outre en ce que** :
- la préparation intensificatrice de décoloration (C) ne contient pas d'eau et contient au moins un peroxyde solide choisi dans le groupe constitué du peroxodisulfate d'ammonium, peroxodisulfate de potassium, peroxodisulfate de sodium, peroxomonosulfate d'ammonium, peroxomonosulfate de potassium, peroxomonosulfate de sodium, peroxodiphosphate de potassium, des peroxodiphosphate de métaux alcalins tels que le peroxodiphosphate de potassium, percarbonate de magnésium, peroxyde de baryum, perborate de sodium, ainsi que du peroxyde d'urée et de peroxyde de mélamine.

4. Kit selon la revendication 3, **caractérisé en ce que** le copolymère ionique non-permanent est contenu dans la préparation de coloration de cheveux (A), à condition que la préparation de coloration de cheveux (A) ne contienne comme composant colorant aucun précurseur de colorant d'oxydation.

5. Kit selon la revendication 3, **caractérisé en ce que** le copolymère ionique non-permanent est contenu dans la préparation oxydante (B).

6. Procédé de coloration de cheveux humains, **caractérisé en ce qu'**on mélange intimement les préparations (A), (B) et (C) des récipients confectionnés séparément (I), (II) et (III) d'un kit multicomposant selon une des revendications 3 à 5, on applique la préparation prête à l'emploi obtenue sur les cheveux, on laisse agir pendant une durée de 2 à 60 minutes, de préférence de 5 à 45 minutes, sur les cheveux, puis on rince les cheveux.
